# EUROPÄISCHE PATENTSCHRIFT

(11) **EP 0 570 681 B1**
(45) Veröffentlichungstag und Bekanntmachung des Hinweises auf die Patenterteilung: **29.04.1998**
(21) Anmeldenummer: 93104319.4
(22) Anmeldetag: 17.03.1993
(51) Int. Cl.: G01N 33/18

(54) **Verfahren zum Nachweis von zersetzungsfähigen organischen Verbindungen in Wasser**
Method for identifying decomposable organic compounds in water
Procédé d'identification de composes organiques décomposables dans l'eau

(30) Priorität: 20.05.1992 DE 4216631
(43) Veröffentlichungstag der Anmeldung: 24.11.1993
(73) Patentinhaber: HÜLS AKTIENGESELLSCHAFT, 45764 Marl (DE)
(72) Erfinder: Duve, Hans, W-4408 Dülmen (DE)

(56) Entgegenhaltungen:
- EP-A- 0 135 685
- EP-A- 0 388 590
- EP-A- 0 498 888
- WO-A-91/06848
- DE-A- 2 823 587
- DE-A- 3 223 167
- GB-A- 2 165 360
- US-A- 3 958 941
- US-A- 4 801 551

## Beschreibung

Die Erfindung betrifft ein Verfahren zum Untersuchen von Wasser, das zersetzungsfähige organische Verbindungen enthält.

Sie verfolgt den Zweck, derartige Verbindungen auch in sehr kleinen Konzentrationen nachzuweisen. Solche Überwachungen sind beispielsweise notwendig für Prozeßwasser, falls darin organische Verbindungen enthalten sein können, die sich auf die Prozeßführung negativ auswirken. Bei dem Prozeßwasser kann es sich z. B. um Kondensat handeln, das aus einer Chemieanlage in das Kraftwerk zurückgeführt wird, oder das als Brauchwasser eingesetzt werden soll.

Die meisten der bekannten Nachweisverfahren für organische Wasserinhaltsstoffe werden nach DIN 38 409 durchgeführt [Deutsche Einheitsverfahren zur Wasser-, Abwasser und Schlamm-Untersuchung. DIN 38 409, Teil 3, Bestimmung des gesamten organischen Kohlenstoffs (TOC) (H.3)]. Bei diesen Verfahren wird eine Wasserprobe angesäuert und der gelöste anorganische Kohlenstoff in Form von Kohlendioxid (CO₂) mit Hilfe eines Stripp-Gases aus der Wasserprobe ausgetragen. Danach wird die Wasserprobe einer Zersetzungsreaktion unterworfen, und das dabei aus den organischen Verbindungen entstehende CO₂ wird gemessen. Daraus wird der Gehalt an organischem Kohlenstoff in der Wasserprobe berechnet. Enthält die zu untersuchende Wasserprobe flüchtige organische Verbindungen, werden diese teilweise oder ganz beim Ausstrippen des anorganischen Kohlenstoffs aus der Wasserprobe entfernt. Dieses führt zu einem Minderbefund bei der Gehaltsbestimmung von organischem Kohlenstoff in der Wasserprobe. Hetero-Atome, z. B. Säurebildner wie Chlor und Schwefel, die bei der Zersetzung von entsprechenden organischen Verbindungen (z. B. Chlorkohlenwasserstoffe, Thioverbindungen) mit entstehen können, werden bei diesen Nachweisverfahren nicht erfaßt.

In DE 32 23 167 wird ein Verfahren zum Untersuchen von Wasser, das zersetzungsfähige Kohlenstoffverbindungen enthält, beschrieben. Mit diesem Verfahren können u. a. in einem entsalzten Wasser mit einer Leitfähigkeit unter o0,1 µS/cm alle Zersetzungsprodukte der durch UV-Bestrahlung zersetzten organischen Wasserinhaltsstoffe nachgewiesen werden.

In EP 0 135 685 wird ein weiteres Verfahren zum Nachweis von organischen Verbindungen in Wasser beschrieben. Bei diesem Verfahren werden alle ionogenen Wasserinhaltsstoffe aus der Wasserprobe entfernt, bevor im UV-bestrahlten Wasser die aus den nichtionogenen organischen Verbindungen entstandenen Zersetzungsprodukte nachgewiesen werden. Beim Entfernen der ionogenen Inhaltsstoffe des Probewasser werden auch alle in ionogener Form vorliegenden organischen Wasserinhaltsstoffe (z. B. Essigsäure oder Diethanol-amin) - vor deren Nachweis - aus dem Wasser ebenfalls entfernt.

Damit stellt sich die Aufgabe, ein Verfahren zu finden, mit dem man sämtliche ionogenen Zersetzungsprodukte, entstanden aus nichtionogenen und ionogenen organischen Verbindungen, in Wasser nachweisen kann, auch wenn dieses Wasser zusätzlich anorganische Ionen enthält.

Die Aufgabe wird erfindungsgemäß zum einen gelöst durch ein Verfahren zum Untersuchen von Wasser, das frei von anorganischen Anionen ist und zersetzungsfähige organische Verbindungen enthält, durch Zersetzen dieser organischen Verbindungen in einem Zersetzer, das gekennzeichnet ist durch:
- Zersetzen der organischen Verbindungen im Zersetzer,
- Entfernen aller Kationen aus dem zu untersuchenden Wasser nach dem Zersetzen der organischen Verbindungen im Zersetzer,
- Nachweisen der Zersetzungsprodukte in dem zu untersuchenden Wasser nach dem Entfernen aller Kationen hinter dem Zersetzer.

Enthält das zu untersuchende Wasser auch anorganische Anionen, wird die Aufgabe erfindungsgemäß zum anderen gelöst durch ein Verfahren zum Untersuchen von Wasser, das neben anorganischen Anionen zersetzungsfähige organische Verbindungen enthält, durch Zersetzen dieser organischen Verbindungen in einem Zersetzer, das gekennzeichnet ist durch:
- Aufteilen des Stromes des zu untersuchenden Wassers vor dem Zersetzer, wobei
- der eine Teilstrom zuerst durch den Zersetzer und anschließend durch einen Kationenaustauscher und danach durch einen lonendetektor geleitet wird, und
- der andere Teilstrom durch einen anderen Kationenaustauscher und einen anderen Ionendetektor geleitet wird, sowie
- Nachweisen der Zersetzungsprodukte durch Differenzbildung der Ionendetektormeßwerte zwischen den Teilströmen.

Das "Entfernen aller Kationen" ist der Austausch aller Kationen im zu untersuchenden Wasser, die keine Wasserstoff-Ionen sind, gegen Wasserstoff-Ionen mittels eines Kationenaustauschers in H-Form.

Als Zersetzer ist bevorzugt ein UV-Reaktor geeignet, wie er z. B. in DE 32 23 167 beschrieben ist. Weiter kann ein thermischer Reaktor als Zersetzer benutzt werden.

Die Zersetzungsprodukte im bestrahlten Wasser können z. B. durch Messen der Leitfähigkeit oder potentiometrisch mittels ionensensitiver Elektroden nachgewiesen werden.

Das zu untersuchende Wasser kann kontinuierlich durch Zersetzer, Kationenaustauscher und Ionendetektor geleitet werden, oder es kann diskontinuierlich in den Zersetzer geleitet werden.

Das zu untersuchende Wasser tritt zuerst in den Zersetzer ein, die Kationen werden hinter dem Zersetzer aus dem zu untersuchenden Wasser entfernt, und die Zersetzungsprodukte werden in dem Wasser nachgewiesen, das den Kationenaustauscher verläßt.

Bei einem Gehalt an anorganischen Ionen im zu untersuchenden Wasser wird neben dem Meßwert im kationenfreien und bestrahlten Wasser parallel dazu der gleichartigen Meßwert im kationenfreien aber unbestrahlten Wasser bestimmt. Durch Differenzwertbildung dieser beiden Meßwerte erhält man den Erhöhungsbetrag des Meßwertes, der sich durch die Zersetzungsprodukte der organischen Verbindungen im zu untersuchenden Wasser ergibt. Dazu wird der Strom des zu untersuchenden Wassers vor dem Zersetzer aufgeteilt, und der eine Teilstrom zuerst durch den Zersetzer und anschließend durch einen Kationenaustauscher und danach durch einen Ionendetektor geleitet. Der andere Teilstrom wird durch einen Kationenaustauscher und danach durch einen weiteren Ionendetektor geleitet, wie in Anspruch 2 beschrieben.

Das erfindungsgemäße Verfahren kann beispielsweise mittels der in der Figur dargestellten Vorrichtung durchgeführt werden.

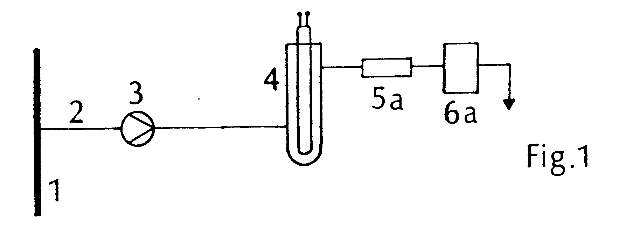

In der Figur 1 wird aus einer Hauptleitung (1) über eine Leitung (2) das zu untersuchende Wasser mit einer Förderpumpe (3) durch einen UV-Reaktor (4) gefahren, in dem sich eine Niederdruck-UV-Lampe befindet. Das den UV-Reaktor (4) verlassende Wasser durchläuft den Kationenaustauscher (5a) und anschließend den Ionendetektor (6a).

Die Meßeinrichtung ist kalibrierfähig, wenn man die Zusammensetzung der im Wasser vorhandenen organischen Verbindungen kennt.

Das erfindungsgemäße Verfahren hat folgende Vorteile:
- Es ermöglicht zersetzungsfähige organische Verbindungen in Wasser nachzuweisen, wobei auch leichtflüchtige organische Verbindungen vollständig erfaßt werden.
- Alle Produkte, die beim Zersetzen der nachzuweisenden organischen Verbindungen entstehen, werden erfaßt.
- Der Nachweis der organischen Verbindungen ist unabhängig von deren elektrischem Ladungszustand möglich.
- Das zu untersuchende Wasser kann neben den organischen Verbindungen auch anorganische Verbindungen enthalten.
- Die organischen Verbindungen können ionogen oder nichtionogen sein.
- Die Zeit zwischen dem Zersetzen der organischen Verbindung und dem Ansprechen des Ionendetektors beträgt nur wenige Sekunden.
- Das Verfahren ist sehr empfindlich und erlaubt den Nachweis von organischen Verbindungen bis hinab zu 1 ppb.
- Das Verfahren ist je nach Fragestellung vielfach variabel und an spezielle Probleme anpaßbar.

Das erfindungsgemäße Verfahren wird an Hand der folgenden Beispiele näher erläutert. In den Beispielen wird der in DE-32 23 167 beschriebene UV-Reaktor als Zersetzer verwendet. Zum Entfernen aller Kationen wird eine Kationenaustauscher-Säule (300 mm hoch, 75 mm Durchmesser) benutzt, die mit einem stark sauren Kationenaustauscher (z. B. LEWATIT S 100 G1) gefüllt ist. Als Ionendetektor dient eine Leitfähigkeitsmeßzelle.

### Beispiel 1: Nachweis eines anionischen organischen Stoffes in salzfreiem Ammoniak-haltigen Wasser.

Das zu untersuchende Wasser enthält keine Salze, jedoch etwa 1 mg/l Ammoniak, das im Wasser als Kation vorliegt, und zunächst keinen ionogenen organischen Stoff Zur Messung des Blindwertes wird das Wasser ein paar Minuten lang mit etwa 5 Liter/Stunde durch eine Vorrichtung nach Figur 1 geleitet. Das den UV-Reaktor verlassende bestrahlte Wasser durchströmt einen Kationenaustauscher und danach eine Leitfähigkeitsmeßzelle, die im vorliegenden Fall eine Leitfähigkeit von 0,2 µS/cm anzeigt.

In den Vorrat an vorgelegtem Wasser wird anschließend eine anionische organische Verbindung gelöst, und zwar 0,20 mg/l Dichloressigsäure, und mit dem Wasser gut gemischt. Beim Durchleiten dieses Wassers durch dieselbe Vorrichtung stellt sich nach Eintritt des die ionogene organische Verbindung enthaltenden Wassers eine Leitfähigkeit von 1,41 µS/cm ein. Damit ist ein ionogener organischer Stoffin salzfreiem Ammoniak-haltigen Wasser unter Erfassen aller seiner Zersetzungsprodukte nachgewiesen worden.

Bei einer vorhergehenden Untersuchung einer Reihe von Proben, die unterschiedliche Konzentrationen von Dichloressigsäure in Reinstwasser enthielten, wurde ein linearer Kalibrierungsfaktor der Leitfähigkeit von 6,44 µS/cm für 1 mg Dichloressigsäure pro Liter Wasser gefunden. Der Erhöhung der Leitfähigkeit von 0,2 µS/cm auf 1,41 µS/cm entspricht die Konzentration von 0,188 mg Dichloressigsäure pro Liter.

### Beispiel 2: Nachweis eines anionischen organischen Stoffes neben Ammoniak in salzhaltigem Wasser.

Das zu untersuchende Wasser aus Beispiel 1 enthält etwa 1 mg/l Ammoniak und 0,20 mg/l Dichloressigsäure. In einen Teil dieses Wassers werden etwa 4 mg/l Salze eingebracht, bestehend aus einer Mischung von MgCl₂, LiBr, CaJ₂, Na₂HPO₄, Ba(NO₃)₂, CUSO₄, Na₂CO₃, und mit dem Wasser gut gemischt. Dieses Wasser wird mit etwa 5 Liter/Stunde durch eine Vorrichtung mit Differenzwertbildung (wie oben beschrieben) geleitet Bei abgeschaltetem Brenner im UV-Reaktor ergeben die Leitfähigkeitsmeßzellen in den beiden Teilströmen praktisch dieselbe Leitfähigkeit von 2,4 µS/cm. Bei eingeschaltetem Brenner im UV-Reaktor steigt die Leitfähigkeit in dem durch den UV-Reaktor geleiteten Teilstrom auf 3,8 µS/cm; in dem anderen Teilstrom bleibt die Leitfähigkeit unverändert. Damit ist ein anionischer organischer Stoff in salzhaltigem Ammoniak-haltigen Wasser nachgewiesen worden.

Aus der Leitfähigkeitserhöhung von 1,2 µS/cm [(3,8 - 2,4 - 0,2) µS/cm] berechnet man mit dem Kalibrierungsfaktor aus Beispiel 1 wiederum eine Konzentration von 0,186 mg Dichloressigsäure pro Liter.

### Beispiel 3: Nachweis eines kationischen organischen Stoffes neben Ammoniak in salzfreiem Wasser.

Das zu untersuchende Wasser enthält keine Salze, jedoch etwa 1 mg/l Ammoniak. Dieses Wasser wird mit etwa 5 Liter/Stunde durch eine Vorrichtung entsprechend Figur 1 geleitet. Die Leitfähigkeitsmeßzelle zeigt in dem bestrahlten Wasser eine Leitfähigkeit von 0,2 µS/cm an.

In den Vorrat an vorgelegtem Wasser wird anschließend eine geringe Menge Diethanolamin gelöst, das in Wasser als Kation vorliegt, und mit dem Wasser gut gemischt. Beim Durchleiten dieses Wassers durch dieselbe Vorrichtung stellt sich nach Eintritt des die ionogene organische Verbindung enthaltenden Wassers eine Leitfähigkeit von 1,50 µS/cm im bestrahlten Wasser ein.

Die Leitfähigkeitserhöhung rührt her von den Zersetzungsprodukten von Diethanolamin, die vom Kationentauscher unverändert durchgelassen werden. Das Ammoniak dagegen wird im Kationenaustauscher zurückgehalten, und zwar unabhängig davon, ob der Brenner im UV-Reaktor eingeschaltet ist oder nicht.

Damit ist ein kationischer organischer Stoff neben Ammoniak in einem salzfreien Wasser nachgewiesen worden.

## Patentansprüche

1. Verfahren zum Untersuchen von Wasser, das frei von anorganischen Anionen ist und zersetzungsfähige organische Verbindungen enthält, durch Zersetzen dieser organischen Verbindungen in einem Zersetzer, gekennzeichnet durch
- Zersetzen der organischen Verbindungen im Zersetzer,
- Entfernen aller Kationen aus dem zu untersuchenden Wasser nach dem Zersetzen der organischen Verbindungen im Zersetzer,
- Nachweisen der Zersetzungsprodukte in dem zu untersuchenden Wasser nach dem Entfernen aller Kationen hinter dem Zersetzer.

2. Verfahren zum Untersuchen von Wasser, das neben anorganischen Anionen zersetzungsfähige organische Verbindungen enthält, durch Zersetzen dieser organischen Verbindungen in einem Zersetzer, gekennzeichnet durch
- Aufteilen des Stromes des zu untersuchenden Wassers vor dem Zersetzer, wobei
- der eine Teilstrom zuerst durch den Zersetzer und anschließend durch einen Kationenaustauscher und danach durch einen lonendetektor geleitet wird, und
- der andere Teilstrom durch einen anderen Kationenaustauscher und einen anderen lonendetektor geleitet wird, sowie
- Nachweisen der Zersetzungsprodukte durch Differenzbildung der Ionendetektormeßwerte zwischen den Teilströmen.

3. Verfahren nach Anspruch 1 oder 2, gekennzeichnet durch
- Zersetzen der organischen Verbindungen in einem UV-Reaktor als Zersetzer

4. Verfahren nach einem der Ansprüche 1 bis 3, gekennzeichnet durch
- kontinuierliches Durchleiten oder diskontinuierliches Einleiten des zu untersuchenden Wassers durch den bzw in den Zersetzer, Kationenaustauscher und Ionendetektor.

## Claims

1. A method for analysing water which is free from inorganic anions and contains decomposable organic compounds, by decomposition of these organic compounds in a decomposer,
characterized by
- decomposition of the organic compounds in the decomposer,
- removal of all cations from the water to be analysed after the decomposition of the organic compounds in the decomposer,
- detection of the decomposition products in the water to be analysed after the removal of all cations downstream of the decomposer.

2. A method for analysing water which, in addition to inorganic anions, contains decomposable organic compounds, by decomposition of these organic compounds in a decomposer,
characterized by
- division of the stream of the water to be analysed upstream of the decomposer,
with
- the one partial stream being passed initially through the decomposer and then through a cation exchanger and thereafter through an ion detector, and
- the other partial stream being passed through another cation exchanger and another ion detector, and
- detection of the decomposition products by subtraction between the ion detector measurements for the partial streams.

3. A method according to either of claims 1 and 2,
characterized by
- decomposition of the organic compounds in a UV reactor as decomposer.

4. A method according to any one of claims 1 to 3,
characterized by
- continuous passage or batchwise introduction of the water to be analysed through or into the decomposer, cation exchanger and ion detector.

## Revendications

1. Procédé d'analyse d'une eau qui est dépourvue d'anions inorganiques et qui contient des composés organiques décomposables, par décomposition de ces composés organiques dans un analyseur,
caractérisé par
- la décomposition des composés organiques dans le désintégrateur,
- l'élimination de tous les cations de l'eau à analyser après décomposition des composés organiques dans le désintégrateur
- la mise en évidence des produits de décomposition dans l'eau à analyser après élimination de tous les cations après le désintégrateur.

2. Procédé d'analyse d'une eau qui contient, outre des anions inorganiques, des composés organiques décomposables, par décomposition de ces composés organiques dans un désintégrateur,
caractérisé par
- la division du courant de l'eau à analyser avant le désintégrateur, opération dans laquelle
- le premier courant partiel est conduit tout d'abord à travers le désintégrateur puis à travers un échangeur de cations et ensuite à travers un détecteur d'ions, et
- l'autre courant partiel est conduit à travers un autre échangeur de cations et un autre détecteur d'ions, ainsi que
- la mise en évidence des produits de décomposition par formation d'une différence entre les valeurs mesurées au détecteur d'ions d'un courant partiel à l'autre.

3. Procédé selon la revendication 1 ou 2,
caractérisé par
la décomposition des composés organiques dans un réacteur à UV comme désintégrateur.

4. Procédé selon l'une des revendications 1 à 3,
caractérisé par
le passage continu ou l'introduction discontinue de l'eau à examiner à travers ou dans le désintégrateur, l'échangeur de cations et le détecteur d'ions.
